# EUROPÄISCHE PATENTSCHRIFT

(11) **EP 1 898 234 B1**
(45) Veröffentlichungstag und Bekanntmachung des Hinweises auf die Patenterteilung: **14.04.2010**
(21) Anmeldenummer: 07112161.0
(22) Anmeldetag: 10.07.2007
(51) Int. Cl.: G01T 1/29, A61B 6/03, A61B 5/055

(54) **Bildgebende medizinische Einheit**
Medical unit producing an image
Unité médicale de radiographie

(30) Priorität: 11.09.2006 DE 102006042572
(43) Veröffentlichungstag der Anmeldung: 12.03.2008
(73) Patentinhaber: SIEMENS AKTIENGESELLSCHAFT, 80333 München (DE)
(72) Erfinder: Rietzel, Eike, 64289, Darmstadt (DE)

(56) Entgegenhaltungen:
- DE-A1- 4 406 996
- DE-A1-102004 049 915
- US-A1- 2003 128 801
- US-A1- 2004 195 512

## Beschreibung

Die Erfindung betrifft eine bildgebende medizinische Einheit mit einem Tomographiegerät zur 3D-Bildgebung und mit einem PET-System zur Positronen-Emissions-Tomographie.

Aus der DE 103 39 493 ist ein PET-CT-Gerät und aus der US 2003/0014 132 ist ein PET-Gerät bekannt.

Bei der Positronen-Emissions-Tomographie (PET) wird dem Patienten ein, durch Beimengung eines Radionuklids mit einer vergleichsweise kurzen Halbwertszeit zu einer Trägersubstanz erhaltener, Tracer, z. B. ¹⁸F-FDG (Flourdeoxyglucose), injiziert, welcher sich in bestimmten Organen und Zellgeweben anreichert und unter Emission von Positronen zerfällt. Bevorzugt erfolgt die Anreicherung in aktiven Krebszellen.

Ein beim radioaktiven Zerfall freigesetztes Positron tritt nach relativ kurzer Distanz von typischerweise einem Millimeter in Wechselwirkung mit einem Elektron, wobei beide Teilchen vernichtet und zwei Gammaquanten mit einer Energie von jeweils 511 keV in diametral entgegengesetzter Richtung abgestrahlt werden. Diese Annihilationsquanten lassen sich in einem das Untersuchungsobjekt bzw. den Patienten umgebenden Detektorring, der eine Vielzahl benachbart angeordneter und einzeln auslesbarer Gammadetektoren umfasst, räumlich und zeitlich aufgelöst nachweisen. Durch eine Koinzidenzkollimierung in einer den Detektoren nachgeschalteten elektronischen Auswerteeinheit lässt sich der Ort der den Zählereignissen jeweils zugrunde liegenden Elektron-Positron-Annihilation auf der gedachten Linie zwischen den signalgebenden Detektorelementen, der so genannten Line of Response, ermitteln. Die Emission der Gammastrahlung erfolgt isotrop, d.h. statistisch gesehen sind alle Richtungen gleich wahrscheinlich. Aus einer statistisch signifikanten Vielzahl von Zählereignissen kann daher die räumliche Häufigkeitsverteilung der radioaktiven Zerfallsprozesse und somit die Verteilung des Tracers im Körper abgeleitet werden. Aus einem derartigen 3D-Volumendatensatz lassen sich weiterhin beliebige zweidimensionale PET-Schnittbilder errechnen.

Bei der PET handelt es sich um eine funktionelle Bildgebung, die vor allem biochemische und physiologische Vorgänge im Organismus abzubilden vermag. Sie erlaubt neben einer guten Analyse des Stoffwechsels insbesondere das Auffinden von Tumoren und Metastasen sowie eine Beurteilung der Perfusion des Herzmuskels. Die PET besitzt aber nur eine relativ schlechte Ortsauflösung (ca. 5 mm), die aus prinzipiellen Gründen ohne zusätzliche Strahlenbelastung nicht mehr gesteigert werden kann. Die PET liefert keine guten anatomischen Bilder, so dass die räumliche Lokalisierung und Zuordnung der erkannten Krankheitsherde Schwierigkeiten bereitet.

Da sich die bei einer PET-Untersuchung injizierten Tracer beispielsweise bevorzugt in Tumoren und Metastasen anreichern und somit deren Lokalisierung erleichtern, kommt insbesondere in der Strahlentherapie vermehrt die Kombination von PET- und CT-Geräten zum Einsatz. Dabei liefert ein CT-Gerät anatomische Information und das PET-Gerät funktionale Information, z.B. über Zellaktivitäten und Stoffwechselvorgänge in einem Zielvolumen. In Kombination lässt sich daraus sowohl geometrische als auch funktionelle Information zur Zielgebietsbestimmung in der Bestrahlungsplanung verwenden.

In der Nachveröffentlichung DE 10 2005 048 853 wird eine Kombination eines PET-Geräts mit einem 3D-Röntgensystem, insbesondere einem Cone-Beam-CT-Gerät, beschrieben. Dabei sind das PET-Gerät und das 3D-Röntgensystem beispielsweise nebeneinander angeordnet, so dass ein auf einem Patiententisch liegender Patient nacheinander in die beiden Bildgebungseinheiten eingebracht werden kann.

Für die Strahlentherapie ist es vorteilhaft, wenn die verwendete Patientenhalterung sowohl in der Bestrahlungsplanung als auch zur Bestrahlung verwendet wird, d.h., dass bevorzugt, baugleiche Patientenhalterungen zum Einsatz kommen. Dies erhöht die erreichbare Präzision bei der Bestrahlung, da mögliche mechanische Ungenauigkeiten in beiden Fällen gleich sind.

In PET-CT-Kombinationsgeräten nach dem Stand der Technik können insbesondere robotische Patientenpositioniereinheiten nicht verwendet werden, die einen Angriffspunkt der Roboterhand beispielsweise unter der Patientenliege aufweisen. Beim Einbringen des Patienten in die "hintere" bildgebende Einheit würde die Roboterhand mit der "vorderen" Bildgebungseinheiten kollidieren. Aus diesem Grund ist es in Kombinationsgeräten nach dem Stand der Technik nicht möglich, derartige Patientenliegen und Patientenpositioniereinheiten für die Bestrahlungsplanung und Bestrahlung einzusetzen.

Eine Aufgabe der Erfindung ist es, ein Kombinations-PET-Tomographie-Gerät anzugeben, das insbesondere die Verwendung derartiger robotischen Patientenpositioniervorrichtungen ermöglicht.

Die Aufgabe wird durch eine bildgebende medizinische Einheit nach Anspruch 1 gelöst. Dabei weist ein Detektorringssystem eines PET-Systems einen Winkelbereich ohne Detektorelemente auf. D.h., das PET-System hat einen offenen und nicht geschlossenen Detektorring. Die Öffnung im Detektorringsystem erlaubt es, ein Patientenhalterungselement, insbesondere einen an einem Patiententisch angreifenden Roboterarm, in die Öffnung einzuführen. Dazu kann der Ring als unterbrochener Ring ausgebildet sein oder eine Aussparung mit einer in ausreichendem Abstand vorgesehenen stützenden Verbindung aufweisen.

Durch das Patientenhalterungselement wird dabei üblicherweise eine Patientenliege bzw. ein Patiententisch in dem PET-System bzw. in dem Tomographie-Gerät zur 3D-Bildgebung positioniert. Patientenhalterungselement und Patientenliege bilden eine Patientenhalterungsvorrichtung. Auf der Patientenliege kann ein Patient liegen. Wenn der Patient nun in der bildgebenden medizinischen Einheit, d.h. in dem Tomographie-Gerät zur 3D-Bildgebung und/oder in dem PET-System positioniert wird, wird das Patientenhalterungselement bei der Positionierung des Patienten zumindest teilweise in die Aussparung des nicht geschlossenen Detektorringsystems gefahren.

Der offene Winkelbereich erlaubt es, dass eine Patientenliege insbesondere auch mit einem robotischen Patientenhalterungssystem bis in das Tomographie-Gerät eingefahren werden kann. Dazu wird aus der Sicht des Patienten das PET-System bevorzugt vor dem Tomographie-Gerät angeordnet. Hinsichtlich der Strahlentherapie ergeben sich die gleichen mechanischen Ungenauigkeiten bei der Positionierung des Patienten sowohl bei der Bildgebung als auch bei der Bestrahlung, so dass sich systematische Fehler verringern lassen. Diese sind in der Strahlentherapie von Nachteil, da sie bei jeder Bestrahlungssitzung auftreten und somit zu einer Abweichung von der geplanten Dosisapplikation führen.

Die bildgebende medizinische Einheit umfasst zusätzlich die Patientenhalterungsvorrichtung zum Einbringen eines Patienten sowohl in das PETals auch in das Tomographie-Gerät. Diese Patientenhalterungsvorrichtung umfasst das Patientenhalterungselement, an dessen Größe die Aussparung des Detektorrings angepasst ist. Hierdurch ist bei der Positionierung des Patienten das Patientenhalterungselement in die Aussparung des nicht geschlossenen Detektorringsystems fahrbar. Vorzugsweise umfasst die Patientenhalterungsvorrichtung weiterhin die Patientenliege, die insbesondere zum Einsatz in einer Strahlentherapieanlage, insbesondere in einer Partikeltherapieanlage, geeignet ist.

In Abhängigkeit des Angriffspunkts an die Patientenliege befindet sich die Öffnung des PET-Systems vorzugsweise unter, seitlich unter oder seitlich neben dem Patiententisch.

In einer vorteilhaften Ausführungsform weist die bildgebende medizinische Einheit ein PET-System mit mindestens einem klappbaren Detektorsegment auf, das ausgebildet ist, um bei einer PET-Untersuchung den offenen Winkelbereich abzudecken und bei einer Röntgenuntersuchung zur Seite zu klappen. Statt klappbar kann ein solches Detektorelement auch schiebbar, oder auf andere Weise in den offenen Winkelbereich einbringbar und entfernbar ausgebildet sein.

Beispielhafte Ausführungsformen für das Tomographie-Gerät sind ein CT-Gerät, Cone-Beam-CT-Gerät oder eine Magnetresonanz-Tomographie-Gerät.

Ferner wird die Verwendung einer bildgebenden medizinischen Einheit nach einem der vorhergehenden Ansprüche für die Gewinnung von Bilddatensätzen für die Strahlentherapieplanung und/oder für die Positionsverifikation bei einer Strahlentherapie beansprucht.

Weitere vorteilhafte Ausführungsformen der Erfindung sind durch die Merkmale der Unteransprüche gekennzeichnet.

Es folgt die Erläuterung von mehreren Ausführungsbeispielen der Erfindung anhand der Figuren 1 bis 5. Es zeigen:
Figur 1 eine Skizze einer bildgebenden medizinischen Einheit mit einem PET-System und einem Tomographie-Gerät,
Figur 2 eine Skizze einer weiteren möglichen bildgebenden medizinischen Einheit mit einem PET-System und einem C-Arm-Röntgen-Gerät,
Figuren 3 und 4 Seitenansicht eines PET-Systems mit klappbaren Detektorelementen im ein- und ausgeklapptem Zustand und
Figur 5 ein PET-System mit einer seitlich angeordneten Öffnung.

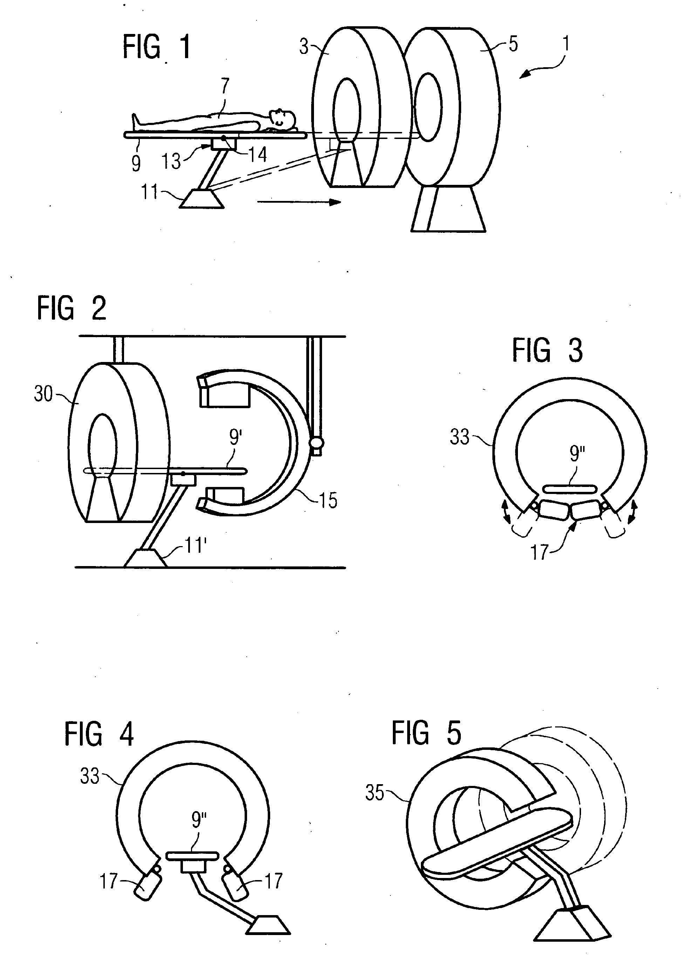

In Figur 1 ist eine bildgebende medizinische Einheit 1 mit einem PET-System 3 und einem Tomographie-Gerät 5, beispielsweise einem Röntgen- oder Magnetresonanz-Computertomographie-Gerät, gezeigt. Das PET-System 3 weist einen Detektorring auf, der nicht ganz geschlossen ist, sondern einen freien Winkelbereich aufweist, der nach unten hin ausgerichtet ist.

Die Öffnung des PET-Geräts 3 erlaubt es, einen Patienten 7, der auf einer Patientenliege 9 liegt, auch mit dem Tomographie-Gerät 5 zu untersuchen, wenn eine Roboterhand einer Patientenhalterung 11 an einem Angriffspunkt 14 über ein Verbindungselement 13 unterhalb der Patientenliege 9 angreift. Beim Einfahren des Patienten 7 in die bildgebende medizinische Einheit 1 kann das Verbindungselement 13 und damit die Roboterhand soweit in die Einheit eingefahren werden, dass sich die Roboterhand in der Öffnung befindet und/oder durch die Öffnung hindurch gefahren werden kann. Somit können auch Roboter, wie sie auch bei der Strahlentherapie zum Einsatz kommen, zur Patientenpositionierung bei der Untersuchung mit der bildgebende medizinische Einheit 1 verwendet werden. Die Öffnung des PET-Geräts ist mindestens so weit, dass der Roboterarm (z.B. ca. 10-20 cm) hindurch passt und/oder maximal so weit, dass die Patientenliege 9 in voller Breite (z.B. ca. 40 -60 cm) hindurch passt. Zur Stabilisierung des Detektorrings kann im offenen Bereich eine Verbindung vorzugsweise auf der dem Tomographiegerät zugeordneten Seite vorgesehen werden. Dabei sollte die Verbindung derart ausgebildet werden, dass sie ausreichend Platz für z.B. die einzubringende Roboterhand - als Verbindungselement - frei lässt.

Figur 2 zeigt einen Aufbau, bei dem das PET-System zusammen mit einem Cone-Beam-CT-Gerät 15 (beispielsweise einem C-Arm-Röntgengerät) verwendet wird. Diese Kombination hat den Vorteil, dass die große Flexibilität des Cone-Beam-CT-Geräts in Kombination mit einem PET-Gerät 30 und einer Roboterpatientenhalterung 11' mit einer Patientenliege 9' eingesetzt werden kann.

Figur 3 zeigt schematisch ein PET-System 33 mit zwei klappbaren Detektorsegmenten 17, die bei Bedarf in die Öffnung des PET-Systems 33 - in Figur 3 beispielhaft unterhalb der Patientenliege 9" - eingebracht werden können. Alternativ könnte nur ein derartiges Segment - alternativ beispielsweise über einen Verschiebemechanismus - eingebracht werden. Bei entsprechend klein dimensionierten Angriffspunkten eines Roboterarms kann ein einziges, z.B. klappbares, Detektorsegment ausreichen, um die Öffnung zu überbrücken. Diese Ausführungsform hat den Vorteil, dass die normalerweise aufgrund der Öffnung fehlende Detektorfläche bei Bedarf ergänzt werden kann. Figur 3 zeigt den geschlossenen Fall, wogegen Figur 4 den Betriebszustand mit zur Seite geklappten Detektorelementen verdeutlicht.

Figur 5 zeigt schematisch ein PET-System 35, bei dem der offene Winkelbereich 19 seitlich angeordnet ist.

## Patentansprüche

1. Bildgebende medizinische Einheit (1) mit einem Tomographie-Gerät (5, 15) zur 3D-Bildgebung und mit einem PET-System (3, 30, 33, 35) zur Positronen-Emissions-Tomographie, wobei das PET-System (3, 30, 33, 35) als ein nicht geschlossenes Detektorringsystem ausgebildet ist, welches eine Aussparung in einem Winkelbereich aufweist, die in ihrer Größe und Form zumindest zum teilweisen Aufnehmen eines Patientenhalterungselements ausgebildet ist, und
wobei zusätzlich eine Patientenhalterungsvorrichtung (11) zum Einbringen eines Patienten in die bildgebende medizinische Einheit (1) vorgesehen ist, welche das Patientenhalterungselement umfasst, derart, dass bei einer Positionierung des Patienten in der bildgebenden medizinischen Einheit (1) das Patientenhalterungselement zumindest teilweise in die Aussparung des nicht geschlossenen Detektorringsystems fahrbar ist.

2. Bildgebende medizinische Einheit (1) nach Anspruch 1, wobei der Winkelbereich im unteren Bereich des Detektorringsystems angeordnet ist, um ein Einfahren einer Patientenliege (9, 9', 9") in die bildgebende medizinische Einheit (1) zu ermöglichen, wobei die Patientenliege (9, 9', 9") an ihrer Unterseite mit dem Patientenhalterungselement gehaltert ist.

3. Bildgebende medizinische Einheit (1) nach Anspruch 1, wobei der Winkelbereich im seitlichen oder seitlich unteren Bereich des Detektorringsystems angeordnet ist, um ein Einfahren einer Patientenliege (9, 9', 9") in die bildgebende medizinische Einheit (1) zu ermöglichen, wobei die Patientenliege (9, 9', 9") seitlich neben oder seitlich unten mit dem Patientenhalterungselement gehaltert ist.

4. Bildgebende medizinische Einheit (1) nach Anspruch 1, wobei die Patientenhalterungsvorrichtung (11, 11') es erlaubt, den Patienten zuerst in das PET-System (3, 30, 33, 35) und dann in das in Einschubrichtung hinter dem PET-System (3, 30, 33, 35) angeordnete Tomographie-Gerät (5, 15) zu bringen.

5. Bildgebende medizinische Einheit (1) nach Anspruch 4, wobei die Patientenhalterungsvorrichtung (11, 11') eine Patientenliege (9, 9', 9") aufweist, die zum Einsatz in einer Strahlentherapieanlage, insbesondere einer Partikeltherapieanlage, geeignet ist.

6. Bildgebende medizinische Einheit (1) nach einem der Ansprüche 4 oder 5, wobei die Patientenhalterungsvorrichtung (11, 11') als robotische Patientenpositioniereinheit ausgebildet ist, welche einen Roboterarm und eine Patientenliege (9, 9', 9") aufweist, wobei ein Angriffspunkt (14) des Roboterarms an der Patientenliege (9, 9', 9") unter der, seitlich unter der oder seitlich neben der Patientenliege (9, 9', 9") angeordnet ist.

7. Bildgebende medizinische Einheit (1) nach einem der Ansprüche 4 bis 6, wobei die robotische Patientenpositioniereinheit ein Verbindungselement (13) aufweist, welches an einem Angriffspunkt (14) an der Patientenliege (9, 9', 9") angreift, wobei das Verbindungselement (13) als Patientenhalterungselement die Größe und Form der Aussparung festlegt.

8. Bildgebende medizinische Einheit (1) nach einem der vorhergehenden Ansprüche, wobei das PET-System (3, 30, 33, 35) mindestens ein einbringbares, z.B. einklappbares oder einschiebbares, Detektor-Segment (17) aufweist, das dazu ausgebildet ist, dass es bei einer PET-Untersuchung den Winkelbereich zumindest teilweise abdeckend und vorzugsweise bei einer Tomographieuntersuchung außerhalb der Aussparung anordbar ist.

9. Bildgebende medizinische Einheit (1) nach einem der vorhergehenden Ansprüche, wobei das Tomographie-Gerät (5, 15) als Röntgen-, Conebeam- oder Magnetresonanz-Computertomograpie-Gerät ausgebildet ist.

10. Verwendung einer bildgebenden medizinischen Einheit (1) nach einem der vorhergehenden Ansprüche für die Gewinnung von Bilddatensätzen für die Strahlentherapieplanung.

## Claims

1. Imaging medical unit (1) having a tomography device (5, 15) for 3D imaging and having a PET system (3, 30, 33, 35) for positron emission tomography, the PET system (3, 30, 33, 35) being designed as a non-closed detector ring system which has a opening in an angular range which in terms of its size and shape is designed at least partially to accommodate a patient support element, and
in addition a patient support apparatus (11) is provided for introducing a patient into the imaging medical unit (1), said patient support apparatus (11) including the patient support element, such that when the patient is positioned in the imaging medical unit (1) the patient support element can be moved at least partially into the opening of the non-closed detector ring system.

2. Imaging medical unit (1) according to claim 1, the angular range being arranged in the lower area of the detector ring system in order to enable a patient couch (9, 9', 9") to be moved into the imaging medical unit (1), the patient couch (9, 9', 9") being supported on its underside by the patient support element.

3. Imaging medical unit (1) according to claim 1, the angular range being arranged in the lateral or laterally lower area of the detector ring system in order to enable a patient couch (9, 9', 9") to be moved into the imaging medical unit (1), the patient couch (9, 9', 9") being supported laterally adjacent or laterally below by the patient support element.

4. Imaging medical unit (1) according to claim 1,
the patient support apparatus (11, 11') permitting the patient to be introduced firstly into the PET system (3, 30, 33, 35) and then into the tomography device (5, 15) arranged in the slide-in direction behind the PET system (3, 30, 33, 35).

5. Imaging medical unit (1) according to claim 4, the patient support apparatus (11, 11') having a patient couch (9, 9', 9") which is suitable for use in a radiation therapy system, in particular a particle therapy system.

6. Imaging medical unit (1) according to one of claims 4 or 5, wherein the patient support apparatus (11, 11') is designed as a robotic patient positioning unit which has a robot arm and a patient couch (9, 9', 9"), a point of engagement (14) of the robot arm being arranged on the patient couch (9, 9', 9") below, laterally below or laterally adjacent to the patient couch (9, 9', 9").

7. Imaging medical unit (1) according to one of claims 4 to 6, the robotic patient positioning unit having a connecting element (13) which engages at a point of engagement (14) on the patient couch (9, 9', 9"), the connecting element (13) as a patient support element determining the size and shape of the opening.

8. Imaging medical unit (1) according to one of the preceding claims, the PET system (3, 30, 33, 35) having at least one detector segment (17) which can be introduced, e.g. can be retracted or inserted, and which is designed so that during a PET examination it is possible to arrange it so that it covers the angular range at least partially and preferably is outside the opening during a tomography examination.

9. Imaging medical unit (1) according to one of the preceding claims, the tomography device (5, 15) being designed as an x-ray, cone beam or magnetic resonance computed tomography device.

10. Use of an imaging medical unit (1) according to one of the preceding claims for obtaining image data records for radiation therapy scheduling.

## Revendications

1. Unité d'imagerie médicale (1) comprenant un appareil de tomographie (5, 15) pour l'imagerie 3D et un système PET (3, 30, 33, 35) pour la tomographie par émission de positrons, ledit système PET (3, 30, 33, 35) étant conçu sous forme d'un système d'anneau détecteur non fermé, comportant dans une zone angulaire une encoche qui en termes de taille et de forme est conçue pour recevoir au moins partiellement un élément de support du patient, et
dans laquelle il est également prévu un dispositif de support du patient (11), destiné à faire entrer un patient dans l'unité d'imagerie médicale (1) et qui comprend ledit élément de support du patient de telle sorte que, lors d'un positionnement du patient dans l'unité d'imagerie médicale (1), ledit élément de support du patient puisse être conduit au moins partiellement dans l'encoche du système d'anneau détecteur non fermé.

2. Unité d'imagerie médicale (1) selon la revendication 1, ladite zone angulaire étant disposée dans la partie basse du système d'anneau détecteur de manière à pouvoir conduire une table (9, 9', 9") recevant le patient dans l'unité d'imagerie médicale (1), ladite table (9, 9', 9") étant supportée au niveau de sa face inférieure par ledit élément de support du patient.

3. Unité d'imagerie médicale (1) selon la revendication 1, ladite zone angulaire étant disposée dans la partie latérale ou latérale basse du système d'anneau détecteur de manière à pouvoir conduire une table (9, 9', 9") recevant le patient dans l'unité d'imagerie médicale (1), ladite table (9, 9', 9") étant supportée latéralement à côté ou latéralement en bas par ledit élément de support du patient.

4. Unité d'imagerie médicale (1) selon la revendication 1, ledit dispositif de support du patient (11, 11') permettant de conduire le patient d'abord dans le système PET (3, 30, 33, 35) puis dans l'appareil de tomographie (5, 15), placé dans le sens de la conduite derrière le système PET (3, 30, 33, 35).

5. Unité d'imagerie médicale (1) selon la revendication 4, ledit dispositif de support du patient (11, 11') comprenant une table (9, 9', 9") recevant le patient, qui est adaptée à être utilisée dans une installation de radiothérapie, en particulier une installation de thérapie par particules.

6. Unité d'imagerie médicale (1) selon l'une des revendications 4 ou 5, ledit dispositif de support du patient (11, 11') étant conçu sous forme d'unité robotique de positionnement du patient, comprenant un bras de robot et une table (9, 9', 9") recevant le patient, un point d'accroche (14) du bras de robot au niveau de ladite table (9, 9', 9") étant placé sous, latéralement sous ou latéralement à côté de la table (9, 9', 9").

7. Unité d'imagerie médicale (1) selon l'une des revendications 4 à 6, ladite unité robotique de positionnement du patient comprenant un élément de jonction (13), s'accrochant dans un point d'accroche (14) au niveau de la table (9, 9', 9"), ledit élément de jonction (13), en tant qu'élément de support du patient, déterminant la taille et la forme de l'encoche.

8. Unité d'imagerie médicale (1) selon l'une des revendications précédentes, ledit système PET (3, 30, 33, 35) comprenant au moins un segment détecteur (17) escamotable, par exemple repliable ou rétractable, conçu pour être disposé pour un examen PET de façon à recouvrir au moins partiellement la zone angulaire, et pour un examen tomographique de préférence à l'extérieur de l'encoche.

9. Unité d'imagerie médicale (1) selon l'une des revendications précédentes, ledit appareil de tomographie (5, 15) étant conçu comme appareil de tomographie par ordinateur à rayons X, à faisceau conique (cone beam) ou à résonance magnétique.

10. Utilisation d'une unité d'imagerie médicale (1) selon l'une des revendications précédentes pour obtenir des ensembles de données d'image en vue de l'établissement du plan de radiothérapie.
